# EUROPEAN PATENT APPLICATION

(11) **EP 4 697 077 A1**
(43) Date of publication of application: **18.02.2026**
(21) Application number: 25193093.9
(22) Date of filing: 31.07.2025
(51) Int. Cl.: G02B 21/00, A61B 1/00, G02B 21/22, G02B 21/36, G02B 25/00, G02B 27/01

(54) **MICROSCOPE FOR MEDICAL DIAGNOSIS AND TREATMENT**

(30) Priority: 14.08.2024 JP 2024135237
(71) Applicant: J. MORITA MANUFACTURING CORPORATION, Fushimi-ku, Kyoto-shi, Kyoto 6128533 (JP)
(72) Inventor: KANZAKI, Yosuke, Kyoto-shi, Kyoto, 612-8533 (JP)
(74) Representative: Müller Hoffmann & Partner

(57) **Abstract**

Provided is a microscope for medical diagnosis and treatment that allows an observer to view a natural stereoscopic image of an object to be observed. The microscope for medical diagnosis and treatment includes an objective optical system, an imaging element, a display element, and an eyepiece optical system configured to direct light of the displayed image from the display element to an observer. The objective optical system includes first and second objective optical systems, the first and second objective optical systems are arranged to form a convergence angle by respective optical axis of the first and second objective optical systems, the number of horizontal or vertical pixels of the display element is 2000 or more and 4000 or less, and an angle of view of the eyepiece optical system is 35 degrees or more and 60 degrees or less.

## Description

### CROSS REFERENCE TO RELATED APPLICATIONS

This nonprovisional application is based on Japanese Patent Application No. 2024-135237 filed on August 14, 2024 with the Japan Patent Office, the entire contents of which are hereby incorporated by reference.

### BACKGROUND

### Field

The present disclosure relates to a microscope used for medical diagnosis and treatment.

### Description of the Background Art

Medical practitioners may observe a patient's site to be diagnosed/treated, using a microscope for medical diagnosis and treatment. In recent years, a 3D digital microscope serving as a medical microscope for observing a patient's site to be diagnosed/treated has been attracting attention, instead of the optical stereo microscope.

For example, Japanese Patent No. 6469292 discloses a system configured to enable an observer to perform dental treatment while stereoscopically viewing a teeth image, by presenting, on a display unit, the teeth image generated by an imaging element provided in a microscope.

### SUMMARY

When an optical stereo microscope is used, an observer can directly observe, through a lens, a site to be diagnosed/treated as it is. When a 3D digital microscope is used, however, an observer observes an image obtained by imaging a site to be diagnosed/treated, instead of directly observing the site through a lens. Therefore, the 3D digital microscope needs to be devised to allow an observer to view a natural stereoscopic image of a site to be diagnosed/treated, as if the observer directly observes the site through a lens. However, there is no microscope for medical diagnosis and treatment devised in such a manner.

An object of the present disclosure is to provide a microscope for medical diagnosis and treatment, capable of allowing an observer to view a natural stereoscopic image of an object to be observed.

According to the present disclosure, a microscope for medical diagnosis and treatment includes: an objective optical system; an imaging element configured to capture an image of a subject formed through the objective optical system; a display element configured to display the image acquired by the imaging element; and an eyepiece optical system configured to direct light of the displayed image from the display element to an observer, the objective optical system includes a first objective optical system and a second objective optical system, the imaging element includes a first imaging element associated with the first objective optical system, and a second imaging element associated with the second objective optical system, the display element includes a first display element associated with the first imaging element, and a second display element associated with the second imaging element, the eyepiece optical system includes a first eyepiece optical system associated with the first display element, and a second eyepiece optical system associated with the second display element, the first objective optical system and the second objective optical system are disposed to form a convergence angle between an optical axis of the first objective optical system and an optical axis of the second objective optical system, the number of horizontal pixels or the number of vertical pixels of the display element is 2000 or more and 4000 or less, and an angle of view of the eyepiece optical system is 35 degrees or more and 60 degrees or less.

The foregoing and other objects, features, aspects and advantages of the present disclosure will become more apparent from the following detailed description of the present disclosure when taken in conjunction with the accompanying drawings.

### BRIEF DESCRIPTION OF THE DRAWINGS

Fig. 1 is a schematic diagram showing a configuration example of a microscope according to the present embodiment as well as a system using the microscope.
Fig. 2 is a block diagram showing a detailed configuration of the microscope.
Fig. 3 is a schematic diagram for illustrating the principle of stereoscopy provided by the microscope.
Fig. 4 is a diagram for illustrating differences between the microscope according to the present embodiment and Comparative Example 1.
Fig. 5 is a diagram showing a configuration of an eyepiece unit.
Fig. 6 is a diagram showing a relationship between an angle of view and the number of pixels.
Fig. 7 is a conceptual diagram for illustrating an apparent distance to a screen for stereoscopic vision.
Fig. 8 is a conceptual diagram for illustrating a principal ray angle.
Fig. 9 is a diagram for illustrating a working distance from an objective unit to a subject.
Fig. 10 is a diagram for illustrating a relationship between a convergence angle and a focus adjustment range.
Fig. 11 is a graph showing the relationship between the convergence angle and the focus adjustment range.
Fig. 12 is a diagram for illustrating a modification.

### DESCRIPTION OF THE PREFERRED EMBODIMENTS

Embodiments of the present disclosure are described in detail with reference to the drawings. The same or corresponding parts in the drawings are denoted by the same reference characters, and a description thereof is not herein repeated.

### <Overall Configuration>

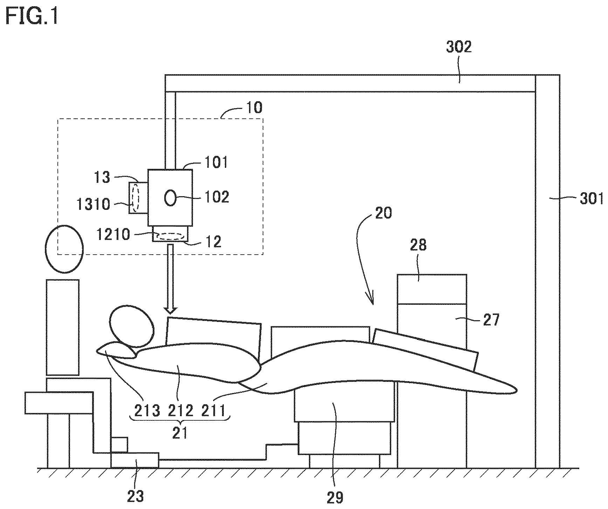

Fig. 1 is a schematic diagram illustrating a configuration example of a microscope 10 according to the present embodiment and a system using microscope 10. An example of the system to which microscope 10 according to the present embodiment is applied is described with reference to Fig. 1. Microscope 10 is a microscope to be used for medical diagnosis and treatment. Dental diagnosis and treatment is presented herein as an example of medical diagnosis and treatment. However, microscope 10 according to the present disclosure is applicable not only to dental diagnosis and treatment but also to diagnosis and treatment in other branches of medicine such as surgery and dermatology.

Fig. 1 shows an example the system including microscope 10 and a chair unit 20. Microscope 10 and chair unit 20 are communicably connected by a CAN (Controller Area Network). Microscope 10 is supported by an arm 302 pivotably attached to a pole 301. Microscope 10 is a digital stereo microscope. Microscope 10 has a function of generating a three-dimensional image of a subject and allowing an observer to view a stereoscopic image of the subject. Arm 302 may be attached to a support member extending from a ceiling or a wall.

Microscope 10 includes a pair of objective units 12, a pair of eyepiece units 13, a pair of handles 102, and a housing 101. In Fig. 1, microscope 10 is illustrated at an angle at which only one objective unit 12, one eyepiece unit 13, and one handle 102 among the pair of objective units 12, the pair of eyepiece units 13, and the pair of handles 102, can be seen. Therefore, the other objective unit 12, the other eyepiece unit 13, and the other handle 102 are not seen in Fig. 1. Housing 101 is attached to the distal end of arm 302. Housing 101 contains the pair of objective units 12 and the pair of eyepiece units 13. The pair of handles 102 is provided on housing 101.

Objective unit 12 includes an objective optical system such as an objective lens 1210, and the eyepiece unit includes an eyepiece optical system such as an eyepiece 1310. In objective unit 12, a portion including objective lens 1210 extends from housing 101, and is directed toward a subject. In eyepiece unit 13, a portion including eyepiece 1310 extends from housing 101, and is directed toward a pupil of an observer.

Microscope 10 generates a pair of images of a subject captured by the pair of objective units 12 to create a pair of images for providing stereoscopic vision. The pair of objective units 12 functions as a photographic device (camera). An observer sees the pair of images with two eyes through the pair of eyepiece units 13. At this time, a stereoscopic image of the subject is presented to the observer.

The observer is, for example, a practitioner. The subject is, for example, a patient. During dental diagnosis/treatment, the subject is the patient's oral cavity. The oral cavity includes teeth, periodontal tissue, tongue, and salivary glands. In order to observe the oral cavity, the practitioner holds handle(s) 102 to move microscope 10 and make fine adjustments of the range to be observed.

Chair unit 20 includes a medical chair 21, a foot controller 23, and a base 29. A patient on medical chair 21 receives medical diagnosis/treatment given by a practitioner. A basin unit 27 is placed around medical chair 21. Chair unit 20 may include basin unit 27. Basin unit 27 includes a cleaning unit 28. Cleaning unit 28 includes a water tap and a spit bowl. The patient uses cleaning unit 28 to rinse the oral cavity. A tool table may be placed around medical chair 21. The tool table may have a cabinet portion for housing multiple types of instruments such as cutting tools and medical tools.

Medical chair 21 includes a seat 211, a backrest 212, and a headrest 213. Seat 211 is attached to base 29. Base 29 has a mechanism for raising and lowering seat 211. Backrest 212 is attached to seat 211 so as to be inclinable with respect to seat 211. Headrest 213 is attached to backrest 212 so as to be inclinable with respect to backrest 212.

Foot controller 23 includes a plurality of pedals that receive a foot-pressing operation by a practitioner. These pedals include a pedal for driving base 29, a pedal for driving backrest 212, and a pedal for driving headrest 213. The practitioner steps on a proper pedal among these pedals so as to change the posture of medical chair 21 to an appropriate position.

Fig. 1 exemplarily shows the position where backrest 212 is substantially horizontal with respect to seat 211 and headrest 213 is substantially horizontal with respect to backrest 212. The position of medical chair 21 is determined by the height of seat 211, the inclination angle of backrest 212, and the inclination angle of headrest 213.

### <Hardware Configuration>

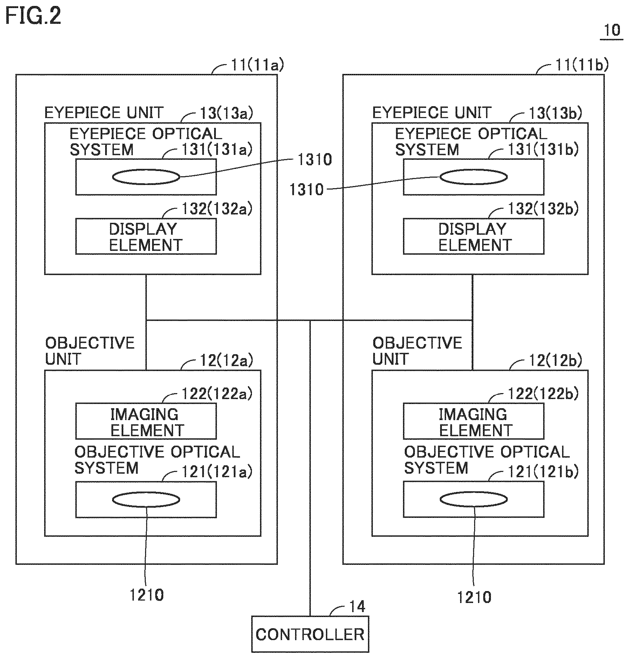

Fig. 2 is a block diagram showing a detailed configuration of microscope 10. As shown in Fig. 2, microscope 10 includes a pair of observation units 11a and 11b and a controller 14. Hereinafter, observation unit 11a and observation unit 11b are referred to collectively as "observation unit 11."

Observation unit 11 includes an objective unit 12 and an eyepiece unit 13. Controller 14 controls objective unit 12 and eyepiece unit 13.

Controller 14 includes a CPU (Central Processing Unit), a RAM (Random Access Memory), and a ROM (Read Only Memory). The CPU executes an operation program stored in the ROM, for example. The ROM stores the program to be executed by the CPU and other data. The RAM serves as a work area for the CPU to execute the program, and temporarily stores the program and data for executing the program, for example. Controllers 14 may be configured by a semiconductor integrated circuit such as at least one processor, at least one ASIC (Application Specific Integrated Circuit), at least one DSP (Digital Signal Processor), at least one FPGA (Field Programmable Gate Array), and/or other circuits having the arithmetic function. Controller 14 may also be configured by arithmetic circuitry (processing circuitry).

The pair of observation units 11 each include objective unit 12 and eyepiece unit 13. Hereinafter, objective unit 12 provided in observation unit 11a is referred to as "objective unit 12a" and objective unit 12 provided in observation unit 11b is referred to as "objective unit 12b." Similarly, eyepiece unit 13 provided in observation unit 11a is referred to as "eyepiece unit 13a" and eyepiece unit 13 provided in observation unit 11b is referred to as "eyepiece unit 13b," hereinafter.

That is, "objective unit 12" is a generic name of "objective units 12a and 12b" and "eyepiece unit 13" is a generic name of "eyepiece units 13a and 13b."

Objective unit 12 includes an objective optical system 121 and an imaging element 122. Eyepiece unit 13 includes an eyepiece optical system 131 and a display element 132. Hereinafter, objective optical system 121 provided in objective unit 12a is referred to as "objective optical system 121a," objective optical system 121 provided in objective unit 12b is referred to as "objective optical system 121b," imaging element 122 provided in objective unit 12a is referred to as "imaging element 122a," and imaging element 122 provided in objective unit 12b is referred to as "imaging element 122b."

That is, "objective optical system 121" is a generic name of "objective optical systems 121a and 121b" and "imaging element 122" is a generic name of "imaging elements 122a and 122b."

Similarly, eyepiece optical system 131 provided in eyepiece unit 13a is referred to as "eyepiece optical system 131a," eyepiece optical system 131 provided in eyepiece unit 13b is referred to as "eyepiece optical system 131b," display element 132 provided in eyepiece unit 13a is referred to as "display element 132a," and display element 132 provided in eyepiece unit 13b is referred to as "display element 132b," hereinafter.

That is, "eyepiece optical system 131" is a generic name of "eyepiece optical systems 131a and 131b" and "display element 132" is a generic name of "display elements 132a and 132b." Display element 132 displays an image acquired by imaging element 122. Eyepiece optical system 131 directs light of the displayed image from display element 132 to an observer.

Imaging element 122 is, for example, a CMOS (Complementary Metal Oxide Semiconductor) image sensor. The shape of an imaging region of imaging element 122 is, for example, a square. The shape of the imaging region of imaging element 122 may not be a square, but may be any of rectangles other than the square. Display element 132 forms a flat panel display such as LCD (Liquid Crystal Display) or organic EL (Electroluminescence). Imaging element 122 captures an image of a subject formed through objective optical system 121.

### <Principle of Stereoscopy>

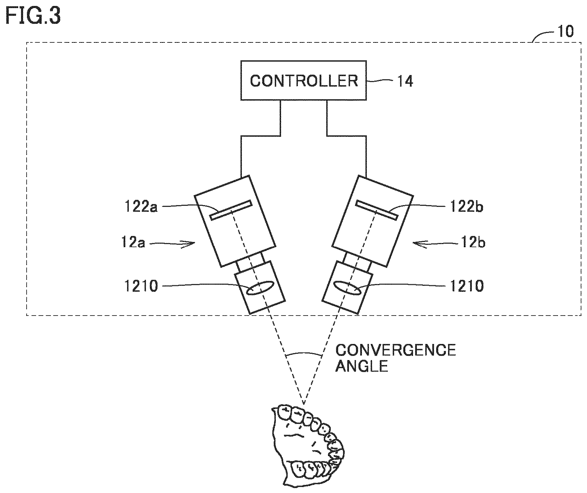

Fig. 3 is a schematic diagram for illustrating the principle of stereoscopy provided by microscope 10. With reference to Fig. 3, the principle of stereoscopy provided by microscope 10 is described.

As shown in Fig. 3, a pair of objective units 12a and 12b is arranged such that a convergence angle is formed at the intersection of respective optical axes passing through respective centers of objective lenses 1210 and 1210. A subject is located at the intersection of the optical axes. Fig. 3 shows the oral cavity of a patient, as an example of the subject. A pair of imaging elements 122a and 122b captures an image of the subject formed by objective lenses 1210, and outputs an image signal to controller 14. In the image acquired by imaging element 122a and the image acquired by imaging element 122b, there is a positional displacement depending on the convergence angle. This positional displacement corresponds to "parallax."

Controller 14 causes the image acquired by imaging element 122a to be displayed on display element 132a (see Fig. 2), and causes the image acquired by imaging element 122b to be displayed on display element 132b (see Fig. 2). As a result, a pair of images causing parallax is displayed on respective display elements 132a and 132b. An observer observes the pair of images through the pair of eyepiece units 13a and 13b shown in Fig. 2. Thus, the observer can stereoscopically view the subject.

### <Comparison between Present Embodiment and Comparative Example 1>

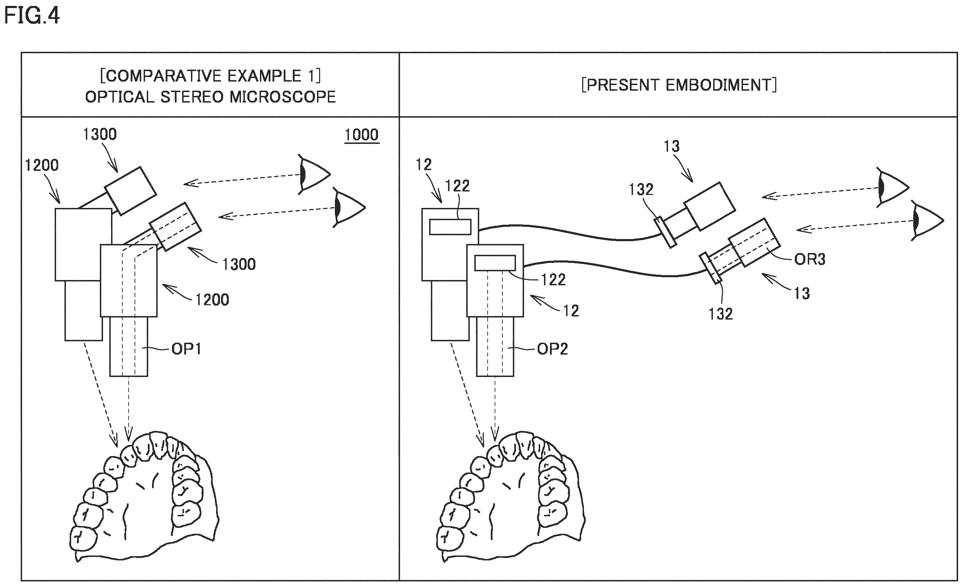

Fig. 4 is a diagram for illustrating differences between microscope 10 according to the present embodiment and Comparative Example 1. A microscope according to Comparative Example 1 is an optical stereo microscope 1000. Optical stereo microscope 1000 includes a pair of objective units 1200 and 1200 and a pair of eyepiece units 1300 and 1300. Microscope 10 according to the present embodiment includes a pair of objective units 12 and 12 and a pair of eyepiece units 13 and 13.

Microscope 10 and optical stereo microscope 1000 have a common feature that these microscopes include a pair of objective units and a pair of eyepiece units. It is obvious that each of objective unit 12 (1200) and eyepiece unit 13 (1300) includes an optical system, the optical system of objective unit 12 (1200) includes an objective lens, and the optical system of eyepiece unit 13 (1300) includes an eyepiece.

Optical stereo microscope 1000 delivers a visual image of an object to be observed, directly to the pupils of an observer, through an optical path OP1 in objective unit 1200 and eyepiece unit 1300. Therefore, the observer can observe the object as it is through the objective lens of objective unit 1200 and the eyepiece of eyepiece unit 1300.

In contrast, microscope 10 captures, by means of imaging element 122, a visual image of the object entering objective unit 12 through an optical path OP2, and displays the image acquired by the imaging, on display element 132 in eyepiece unit 13. An observer observes the visual image delivered from display element 132 through an optical path OR3. Thus, microscope 10 is a "3D digital microscope."

In the case of the 3D digital microscope, the objective units and the eyepiece units can be operated independently of each other, and therefore, the degree of freedom of the posture taken for receiving treatment can be improved.

Further, in the case of optical stereo microscope 1000, when a laser beam used for dental diagnosis/treatment enters the objective lens, there is a risk that the laser beam directly enters the pupils of the observer. In contrast, in the case of the 3D digital microscope, there is no such risk. Thus, the 3D digital microscope has numerous advantages that the optical stereo microscope 1000 does not have.

However, the 3D digital microscope has a problem of how to allow an observer to stereoscopically view an object in a natural condition (as it is), by devising the imaging and the way to present the image. In short, it is still a problem for the "3D digital microscope" to allow an observer to stereoscopically view an natural image of an object. Comparative Example 1 in which a visual image of an object itself is provided to an observer through the lens does not have such a problem. In connection with the present embodiment, more specific design values and the like required for the 3D digital microscope in order to solve this problem are described with reference to microscope 10 as an example.

### <Configuration of Eyepiece Unit>

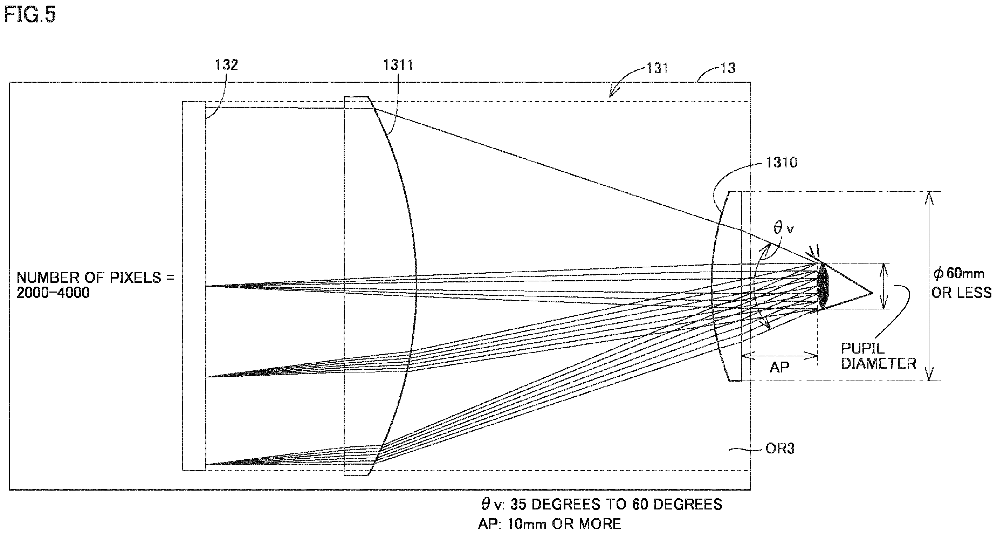

Fig. 5 is a diagram showing a configuration of eyepiece unit 13. As shown in Fig. 5, eyepiece unit 13 includes a display element 132 and an eyepiece optical system 131. Eyepiece optical system 131 includes an eyepiece 1310 and a field lens 1311. An observer looks through eyepiece 1310 to observe a visual image displayed on display element 132. In eyepiece unit 13, an optical path OR3 for directing the image displayed on display element 132 to the pupil of the observer is formed.

Eyepiece 1310 and field lens 1311 are disposed in optical path OR3. Field lens 1311 is placed between display element 132 and eyepiece 1310. As described above, eyepiece optical system 131 includes a plurality of lenses arranged in the direction of optical path OR3 that directs light of the displayed image from display element 132 to an observer. Aberrations are corrected by combining field lens 1311 and eyepiece 1310.

If only one eyepiece 1310 is placed in optical path OR3, eyepiece 1310 functions like a so-called "magnifying glass." In this case, although substantially no distortion occurs in the visual image at the center of the field of view, the image is distorted or blurred in the periphery of the field of view, due to the influence of aberration. Therefore, in the present embodiment, a plurality of lenses are disposed in optical path OR3 so that a visual image with little distortion in the entire field of view can be provided to the observer. In Fig. 5, two lenses are shown as an example of the plurality of lenses. However, three or more lenses may be disposed in optical path OR3.

Display element 132 forms a screen for displaying a visual image. Light of the displayed image from the center of the screen passes through the center of field lens 1311 and the center of eyepiece 1310 to reach the pupils of the observer. Light of the displayed image from an end of the screen is refracted by field lens 1311 and thereafter reaches the pupils of the observer through eyepiece 1310. The observer feels that the screen on which the image is displayed is spread across the range of an angle of view θv.

The field lens 1311 also contributes to reduction of the size of eyepiece 1310. In the case where field lens 1311 is not placed between display element 132 and eyepiece 1310, it is necessary to increase the size of eyepiece 1310 so that light of the displayed image from one end to the other end of the screen can be incident. However, field lens 1311 can be placed between display element 132 and eyepiece 1310, to enable the optical axis to be bent in the direction toward the center of eyepiece 1310. As a result, the diameter of eyepiece 1310 can be reduced.

### <Design Values for Eyepiece Unit 13>

Design values for eyepiece unit 13 are described. As shown in Fig. 5, an eyepoint AP of eyepiece unit 13 is 10 mm (millimeters) or more, the angle of view θv of the eyepiece optical system in eyepiece unit 13 is 35 degrees or more and 60 degrees or less, the diameter of eyepiece 1310 is 60 mm or less, and the number of pixels of display element 132 is 2000 or more and 4000 or less. The design value of the number of pixels may be a design value for the number of horizontal pixels or a design value for the number of vertical pixels. For example, in the case where the field of view is designed to have the shape of a perfect circle, the number of horizontal pixels and the number of vertical pixels are equal to each other. In order to design the field of view in the shape of a perfect circle, the designer needs to employ imaging element 122 having a square imaging region. In the case where imaging element 122 having a shape of any of rectangles other than square is employed, the field of view is a circle whose diameter is the vertical dimension.

The eyepoint AP is the distance from a lens surface, on the observer side, of eyepiece 1310, to the pupil of the observer. Generally, in the case where the eyepoint AP is short (e.g., 5 mm), the eyepoint AP will not affect observation by the observer with naked eyes. However, in the case where the eyepoint AP is short, the observer wearing glasses needs to remove the glasses and look into eyepiece 1310.

A design value of 10 mm corresponds approximately to the distance from glasses to the observer's pupil. Therefore, the eyepoint AP can be designed to be 10 mm or more to allow an observer wearing glasses to easily look into eyepiece 1310. A more preferable design value of the eyepoint AP is 17 mm or more and 20 mm or less.

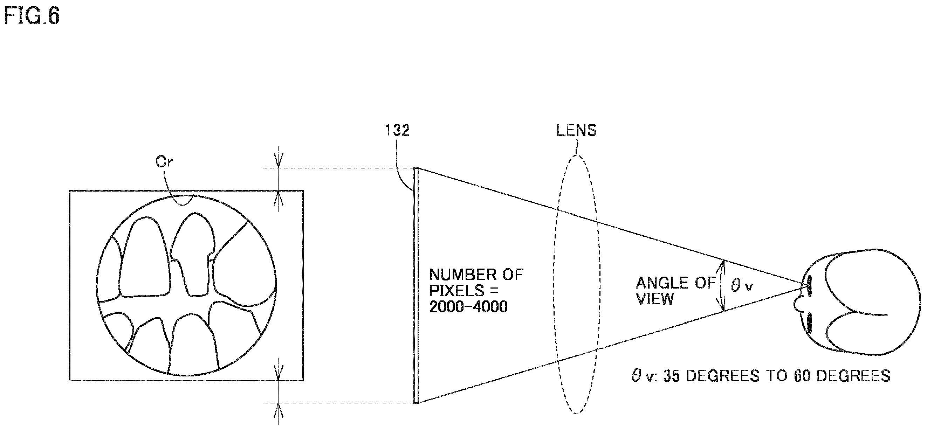

Fig. 6 is a diagram showing a relationship between the angle of view and the number of pixels. The relationship between the angle of view θv and the number of pixels of display element 132 is described in detail with reference to Fig. 6. Angle of view refers to the angle of a field of view of an observer when looking through eyepiece 1310. When the field of view is too wide, the number of pixels per angle of view of 1 degree is smaller and accordingly pixels are conspicuous. Therefore, when the angle of view is too wide, the observer feels that the resolution of the visual image is low. In contrast, when the angle of view is too narrow, the observer can see only a small part of the object to be observed, and accordingly the work efficiency of the observer is degraded.

Here, the unit "ppd (pixels per degree)" is used to more specifically describe the relationship between the angle of view and the pixels. "ppd" means the number of pixels of a screen in the horizontal or vertical direction, included in an angle of view of 1 degree. For example, 60 ppd corresponds to the limit value of the resolution at which an observer with a visual acuity of 1.0 can identify one pixel. Therefore, when an observer with a visual acuity of 0.7 views a 60 ppd screen, the observer cannot clearly identify the boundary between two pixels adjacent to each other, and feels that the boundary between the two adjacent pixels is blurred. As a result, the observer with a visual acuity of 0.7 can see a continuous visual image in which there is no boundary between pixels.

The inventor has conducted an experiment to find that when a design value of 40 ppd or more is adopted, an observer having a visual acuity of about 1.0 to 1.2 can recognize boundaries between pixels, but feels that a natural and clear visual image in which the boundaries are almost unnoticeable is displayed on the screen.

The number of horizontal pixels or the number of vertical pixels of display element 132 is 2000 or more and 4000 or less, and the angle of view θv of eyepiece optical system 131 is 35 degrees or more and 60 degrees or less. Examples of the combination of the number of pixels and the angle of view in the above-specified range of the number of pixels and the above-specified range of the angle of view are indicated below together with the values of "ppd".
(A) (2000 pixels, angle of view: 60 degrees), 33 ppd
(B) (2000 pixels, angle of view: 35 degrees), 57 ppd
(C) (4000 pixels, angle of view: 60 degrees), 60 ppd
(D) (4000 pixels, angle of view: 35 degrees), 114 ppd

Among (A) to (D), (B) to (D) satisfy a condition of "40 ppd or more." It is therefore desirable that the designer adopts any one of (B) to (D) among (A) to (D), as design values (the number of pixels and the angle of view) for microscope 10. The designer, however, may adopt (A) as design values for microscope 10, although (A) does not satisfy the condition of "40 ppd or more."

As shown in Fig. 6, eyepiece optical system 131 forms an image circle Cr whose maximum diameter is smaller than the horizontal dimension of display element 132. The image circle Cr shown in Fig. 6 may be a perfect circle. In this case, eyepiece optical system 131 forms an image circle Cr whose maximum diameter is smaller than the horizontal dimension and the vertical dimension of display element 132. The image circle Cr may be an ellipse. In this case, the maximum diameter of the image circle Cr may be smaller than the horizontal dimension of display element 132 and/or smaller than the vertical dimension of display element 132.

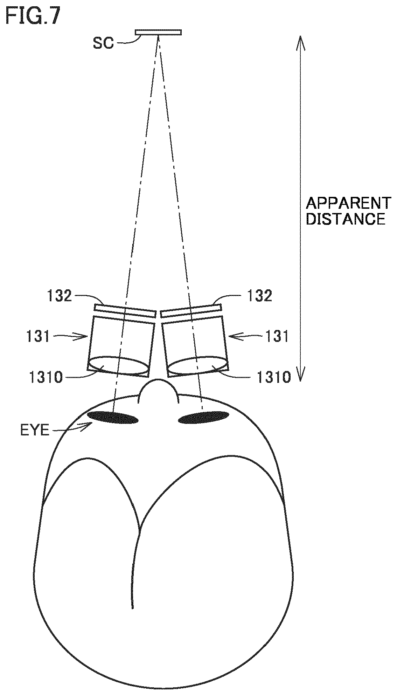

Fig. 7 is a conceptual diagram for illustrating an apparent distance to a screen SC for stereoscopic vision. When stereoscopically viewing an object, an observer gazes one point on the object with each of the left and right eyes oriented inward. Since the human brain unconsciously controls the distance to the object and respective orientations of the left and right eyes, usually the observer can stereoscopically view the object without feeling uncomfortable. However, when the relationship between the distance to the object and the convergence angle of the eyes is unnatural, which is significantly different from the usual relationship, "convergence angle inconsistency" occurs. When the convergence angle inconsistency occurs, the observer feels great fatigue in the eyes, although the observer can stereoscopically view the object.

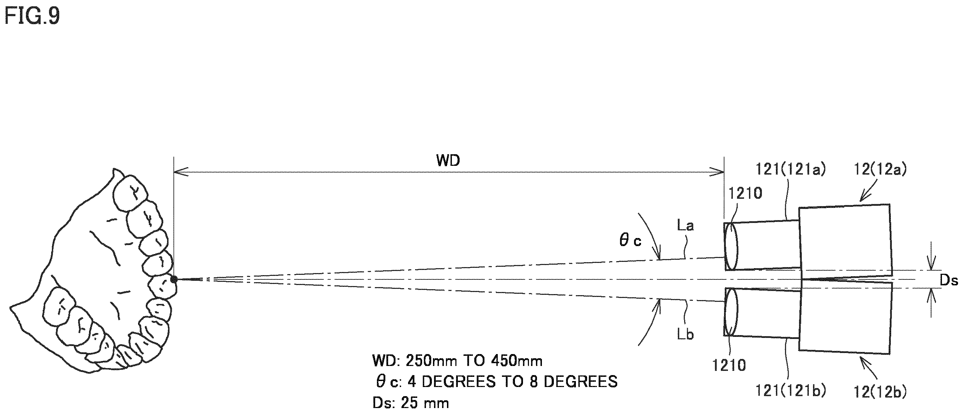

Moreover, when a visual image is located at a close distance such as about 30 mm from the eyes of the observer, the act of observing the visual image itself causes fatigue. Therefore, in the present embodiment, the "apparent distance" to the screen SC is adjusted to about 250 mm by using eyepiece 1310, and the convergence angle is set to an angle appropriate for the pair of eyepiece optical systems 131 so that the line of sight of the left eye and the line of sight of the right eye of the observer coincide with each other at the position located 250 mm ahead. Thus, the eyepiece optical system capable of providing natural stereoscopic vision causing little fatigue can be established. A specific example of the convergence angle is described later herein with reference to Fig. 9.

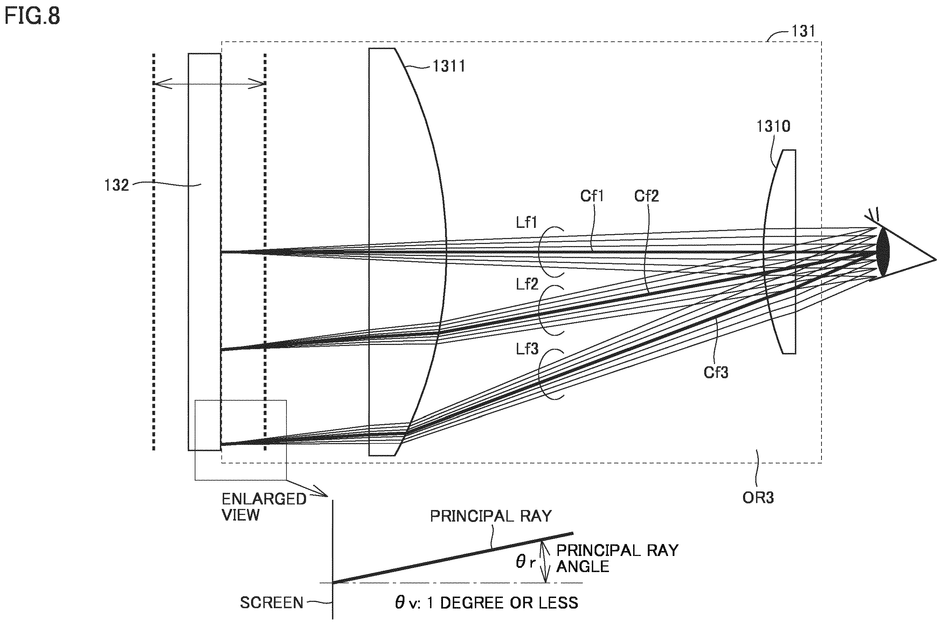

Fig. 8 is a conceptual diagram for illustrating a principal ray angle θr. In eyepiece optical system 131, a plurality of light beams from a screen of display element 132 toward the pupils of an observer are generated. Fig. 8 shows light beams Lf1 to Lf3 as an example of the plurality of light beams. A ray of light generated in the center of a light beam is referred to as "principal ray." Fig. 8 shows principal rays Cf1 to Cf3.

The principal ray angle θr is an angle formed by the principal ray and the normal line to the screen of display element 132. The principal ray angle θr of microscope 10 according to the present embodiment has a value of 1 degree or less. In the following, a reason why the principal ray angle θr is designed to be a value of 1 degree or less is described.

Microscope 10 adjusts the visibility by changing the distance from display element 132 to eyepiece 1310 depending on the visual acuity of the observer. For example, controller 14 (see Fig. 2) horizontally moves display element 132 in the direction of the optical path OR, in response to operation by the observer. When the principal ray angle θr is large, the range of the screen that the observer can view after the visibility adjustment is different from the one before the visibility adjustment. In other words, when the principal ray angle θr is large, the magnification of eyepiece optical system 131 after the visibility adjustment is different from the one before the visibility adjustment.

Particularly in the case where respective visual acuities of the right and left eyes of the observer are greatly different from each other, the difference in magnification between the right and left eyepiece optical systems 131 is accordingly large. It is therefore difficult for the observer to stereoscopically view an object. According to an experiment conducted by the inventor, in order to adjust the visibility to fall within a range satisfying the JIS standard, it was necessary to design the range of movement of display element 132 to be about 28 mm. When the principal ray angle was 1 degree, the region of the screen that the observer could visually recognize changed by about 0.5 mm after the visibility adjustment, as compared with the one before the visibility adjustment. One pixel of the display element used for the experiment was 0.024 mm. Therefore, in the case where the principal ray angle is 1 degree, the region of the screen that the observer can visually recognize changes by a screen region corresponding to about 21 pixels after the visibility adjustment, as compared with the one before the visibility adjustment.

The inventor has confirmed through experiments that when the amount of change in the number of pixels caused by the visibility adjustment is about 20, substantially the observer does not feel uncomfortable. In view of this, the inventor has concluded that it is appropriate to set the principal ray angle θr to 1 degree or less.

### <Design Values for Objective Unit 12>

Fig. 9 is a diagram for illustrating a working distance WD from objective unit 12 to a subject. Fig. 9 shows an oral cavity of a patient, as an example of the subject. As shown in Fig. 9, objective optical system 121a and objective optical system 121b are arranged so as to form a convergence angle θc by the optical axis of objective optical system 121a and the optical axis of objective optical system 121b.

The working distance WD of objective optical system 121 is 250 mm or more and 450 mm or less, the convergence angle θc of objective optical system 121 is 4 degrees or more and 8 degrees or less, and the distance Ds between the pair of objective optical systems 121 and 121 is 25 mm. The working distance WD is the distance from objective optical system 121 to the subject. More specifically, the working distance WD is the distance to the subject, from the lens surface, on the subject side, of objective lens 1210.

In a general microscope, the working distance is about several millimeters. Even in the case of a general optical stereo microscope, the working distance is about 50 mm. Thus, the working distance of the general microscope is short. However, in the case of the microscope for medical diagnosis and treatment, an observer needs to insert a medical tool (such as air turbine) into a site to be diagnosed/treated while enlarging the site with the microscope. In consideration of the ease of operation by the observer, the working distance WD is preferably long in the case of the microscope for medical diagnosis and treatment. Therefore, in the present embodiment, the working distance WD is designed to be 250 mm or more and 450 mm or less.

When the working distance WD is set to 250 mm or more, the diameter of objective lens 1210 is about 25 mm. From the viewpoint of compactness, it is desirable to reduce the diameter of objective lens 1210, however, when the diameter is reduced, the F-number (aperture value) is reduced. As a result, the illuminance of the optical system is significantly decreased. Therefore, in the present embodiment, the diameter of objective lens 1210 is set to about 25 mm.

When the diameter of objective lens 1210 is about 25 mm, the distance Ds between objective optical system 121 (121a) and objective optical system 121 (121b) needs to be at least 25 mm in consideration of the imaging function. It should be noted that he distance between respective objective lenses 1210 provided in the pair of objective optical systems 121 may be defined as "distance Ds."

When the working distance WD is set to 250 mm and the diameter of objective lens 1210 is set to 25 mm, the convergence angle θc is about 5.7 degrees. The longer the distance Ds, the larger the convergence angle θc. For example, when the working distance WD is set to 250 mm and the distance Ds is set to 35 mm, the convergence angle θc is about 8 degrees. From the viewpoint of the diameter of objective lens 1210, it is desirable to set the convergence angle θc to about 5.7° or more. It should be noted that the working distance WD may be 250 mm or more, and may exceed 450 mm, for example.

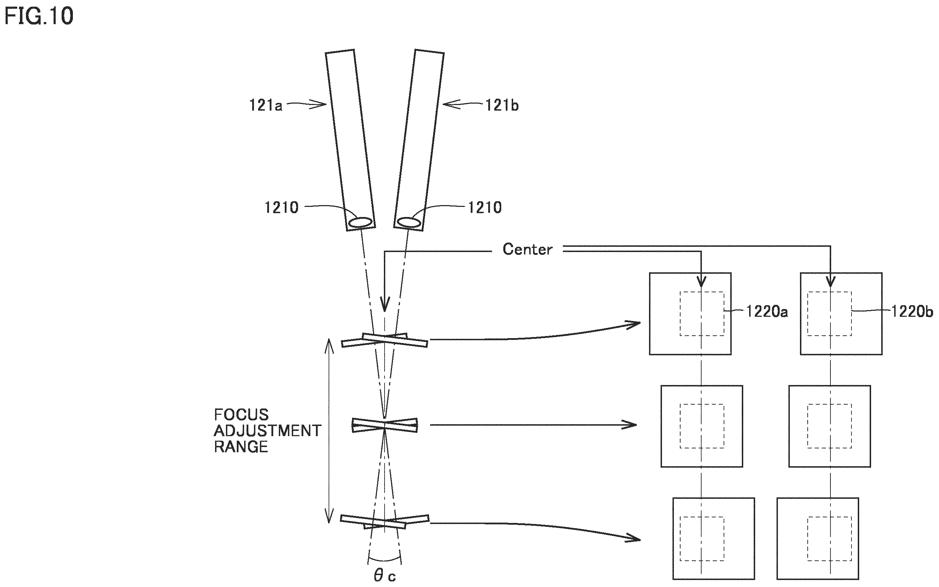

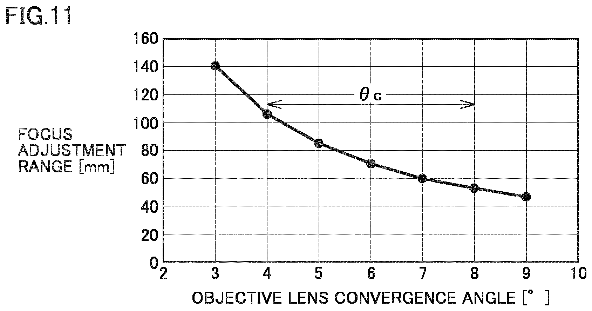

Fig. 10 is a diagram for illustrating a relationship between the convergence angle θc and a focus adjustment range. Fig. 11 is a graph showing the relationship between the convergence angle θc and the focus adjustment range. As shown in Fig. 10, imaging ranges 1220a and 1220b captured by objective optical systems 121a and 121b deviate depending on the focal distance. When the "Center" line portions shown in Fig. 10 are included respectively in both of imaging range 1220a and imaging range 1220b, microscope 10 can acquire a pair of images for stereoscopic vision.

Fig. 11 is a graph obtained when the working distance WS is set to 350 mm and the magnification of objective lens 1210 is set to ×33. As shown in the graph, it is seen that as the convergence angle θc is set smaller, a wider focus range can be obtained. In consideration of practicality, it is desirable to set the convergence angle θc to 4 degrees or more and 8 degrees or less.

### <Modification>

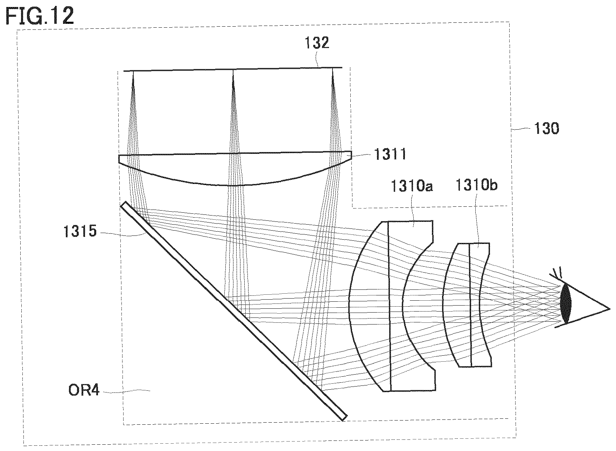

Fig. 12 is a diagram for illustrating a modification. An eyepiece unit 130 according to the modification differs from eyepiece unit 13 described above in that the former is provided with a mirror 1315 and two eyepieces (eyepieces 1310a and 1310b).

In eyepiece unit 130, a field lens 1311 and eyepieces 1310a and 1310b are arranged in such a manner that the optical axis of field lens 1311 intersects with respective optical axes of eyepieces 1310a and 1310b. Mirror 1315 refracts light of the displayed image toward an observer. More specifically, mirror 1315 reflects the light of the displayed image output from field lens 1311 to change the direction of travel of the light of the displayed image in such a manner that causes the light of the displayed image to travel toward eyepieces 1310a and 1310b. Therefore, an optical path OR4 of eyepiece unit 130 is bent from the direction parallel to the optical axis of field lens 1311 to the direction parallel to the optical axes of eyepieces 1310a and 1310b.

According to the modification, the size of eyepiece unit 130 can be reduced in the direction parallel to the optical axes of eyepieces 1310a and 1310b. Accordingly, the observer can observe a site to be diagnosed/treated while looking through eyepiece unit 130, at a position close to the patient. Further, eyepiece unit 130 according to the modification is provided with two eyepieces 1310a and 1310b. Therefore, the resolution can be further improved as compared with eyepiece unit 13. Thus, the eyepiece optical system according to the modification includes a plurality of lenses (field lens 1311 and eyepieces 1310a and 1310b) arranged in the direction of the optical path OR4 that directs light of the displayed image from display element 132 to the observer.

Eyepiece unit 130 is provided with field lens 1311, similarly to eyepiece unit 13. Therefore, as described above, the diameters of eyepieces 1310a and 1310b can be reduced. Further, in eyepiece unit 130, field lens 1311 enables reduction of the size of mirror 1315.

The modification provides a microscope 10 configured by applying eyepiece unit 130 instead of eyepiece unit 13. The foregoing describes various design values for eyepiece unit 13. These design values may also be applied to eyepiece unit 130 according to the modification, as long as the design values cause no inconsistency.

### <Other Modifications>

The present embodiment presents an example in which objective unit 12 and eyepiece unit 13 are housed in observation unit 11. However, there may be no observation unit 11 that houses objective unit 12 and eyepiece unit 13. It is at least necessary for microscope 10 to have a structure for transmitting an image acquired by objective unit 12 to eyepiece unit 13.

Therefore, it is at least necessary that objective unit 12 and eyepiece unit 13 are communicably connected to each other. In this case, a configuration for allowing objective unit 12 and eyepiece unit 13 to directly communicate with each other may be employed, or a configuration for allowing objective unit 12 and eyepiece unit 13 to communicate with each other through controller 14 may be employed. As a communication method, wired communication for which physical conductors are used may be employed, or wireless communication for which no physical conductor is used may be employed.

The present embodiment illustrates specific examples of various design values for microscope 10. However, it may be at least necessary for microscope 10 that the number of horizontal pixels or the number of vertical pixels of display element 132 is 2000 or more and 4000 or less, and the angle of view of eyepiece optical system 131 is 35 degrees or more and 60 degrees or less. Under such preconditions, it may be at least necessary for microscope 10 to employ at least one of the other design values described in connection with the present embodiment.

It should be construed that the embodiments disclosed herein are given by way of illustration in all respects, not by way of limitation. It is intended that the scope of the present disclosure is defined by claims, not by the description above, and encompasses all modifications equivalent in meaning and scope to the claims. It should be noted that the features illustrated in connection with the embodiments and those illustrated in connection with their modifications may be combined as appropriate.

## Claims

1. A microscope for medical diagnosis and treatment, comprising:
an objective optical system;
an imaging element configured to capture an image of a subject formed through the objective optical system;
a display element configured to display the image acquired by the imaging element; and
an eyepiece optical system configured to direct light of the displayed image from the display element to an observer, wherein
the objective optical system includes a first objective optical system and a second objective optical system,
the imaging element includes a first imaging element associated with the first objective optical system, and a second imaging element associated with the second objective optical system,
the display element includes a first display element associated with the first imaging element, and a second display element associated with the second imaging element,
the eyepiece optical system includes a first eyepiece optical system associated with the first display element, and a second eyepiece optical system associated with the second display element,
the first objective optical system and the second objective optical system are disposed to form a convergence angle between an optical axis of the first objective optical system and an optical axis of the second objective optical system,
the number of horizontal pixels or the number of vertical pixels of the display element is 2000 or more and 4000 or less, and
an angle of view of the eyepiece optical system is 35 degrees or more and 60 degrees or less.

2. The microscope for medical diagnosis and treatment according to claim 1, wherein the eyepiece optical system includes a plurality of lenses arranged in a direction of an optical path that directs the light of the displayed image from the display element to the observer.

3. The microscope for medical diagnosis and treatment according to claim 2, wherein the plurality of lenses include:
an eyepiece; and
a field lens placed between the display element and the eyepiece.

4. The microscope for medical diagnosis and treatment according to claim 1 or 2, wherein the eyepiece optical system includes an eyepiece having a diameter of 60 millimeters or less.

5. The microscope for medical diagnosis and treatment according to any one of claims 1 to 4, wherein
the convergence angle is 4 degrees or more and 8 degrees or less, and
a working distance is 250 millimeters or more.

6. The microscope for medical diagnosis and treatment according to any one of claims 1 to 5, wherein the eyepiece optical system is configured to form an image circle having a maximum diameter smaller than a horizontal dimension or a vertical dimension of the display element.

7. The microscope for medical diagnosis and treatment according to any one of claims 1 to 6, wherein a principal ray angle of the eyepiece optical system is 1 degree or less.

8. The microscope for medical diagnosis and treatment according to any one of claims 1 to 7, wherein an eyepoint of the eyepiece optical system is 10 millimeters or more.

9. The microscope for medical diagnosis and treatment according to any one of claims 1 to 8, wherein the eyepiece optical system includes a mirror configured to refract the light of the displayed image toward the observer.

10. The microscope for medical diagnosis and treatment according to claim any one of claims 1 to 9, wherein a distance between the first objective optical system and the second objective optical system is 25 mm.
